# FASCICULE DE BREVET EUROPEEN

(11) **EP 0 703 781 B1**
(45) Date de publication et mention de la délivrance du brevet: **10.05.2000**
(21) Numéro de dépôt: 94919712.3
(22) Date de dépôt: 15.06.1994
(51) Int. Cl.: A61K 31/415

(54) **UTILISATION DE L'EFAROXAN ET DE SES DERIVES POUR LA PREPARATION D'UN MEDICAMENT DESTINE AU TRAITEMENT DE LA MALADIE DE PARKINSON**
VERWENDUNG VON EFAROXAN UND DESSEN DERIVATEN ZUR HERSTELLUNG EINES ARZNEIMITTELS ZUR BEHANDLUNG PARKINSONSCHER KRANKHEIT
USE OF EFAROXAN AND DERIVATIVES THEREOF FOR THE PREPARATION OF A MEDICAMENT FOR THE TREATMENT OF PARKINSON'S DISEASE

(30) Priorité: 18.06.1993 FR 9307379
(43) Date de publication de la demande: 03.04.1996
(73) Titulaire: PIERRE FABRE MEDICAMENT, 92100 Boulogne (FR)
(72) Inventeur: COLPAERT, Francis, F-81100 Castres (FR); BRILEY, Michael, F-81100 Castres (FR); IMBERT, Thierry, F-81290 Viviers-les-Montagnes (FR)
(74) Mandataire: Ahner, Francis
(86) Numéro de dépôt international: FR9400715
(87) Numéro de publication internationale: WO9500145

(56) Documents cités:
- EP-A- 0 071 368
- EP-A- 0 486 385
- BRAIN RES., vol.562, no.2, 25 Octobre 1991 pages 216 - 224 M.MAVRIDIS ET AL. 'Differential modulation of (+)-amphetamine-induced rotation in unilateral substantia nigra-lesioned rats by alpha1 as compared to alpha2 agonists and antagonists'
- NEUROLOGY, vol.41, no.7, Juillet 1991 pages 986 - 991 J.GHIKA ET AL. 'Idazoxan treatment in progressive supranuclear palsy' cité dans la demande
- J.MED.CHEM., vol.27, no.5, 1984 pages 570 - 576 C.B.CHAPLEO ET AL. 'Alpha-Adrenoreceptor Reagents. 2. Effects of Modification of the 1,4-Benzodioxan Ring System on alpha-Adrenoreceptor Activity'
- ARCH.INT.PHARMACODYN.THER., vol.277, no.2, 1985 pages 180 - 191 G.JOLY ET AL. 'Antagonistic Effects of S9871 or (imidazolinyl-2)-2-dihydro 2,3 benzofurane and its Stereoisomers on Some Central and Peripheral Actions of alpha2-Agonists'

## Description

La présente invention concerne l'utilisation de l'Efaroxan et ses dérivés, pour la préparation d'un médicament destiné au traitement de la maladie de Parkinson.

La maladie de Parkinson est une maladie neurodégénérative affectant particulièrement les neurones de la substance noire - pars compacta - et ses projections nigrostriées. Les manifestations symptomatiques sont des troubles moteurs tels que tremblements, rigidité musculaire, hypokinésie. Le diagnostic de la maladie est délicat, seule une analyse histologique pratiquée post-mortem, par la mise en évidence de dégénérescence cellulaire au niveau de la substance noire permet d'affirmer sans ambiguité le diagnostic. Cette dégénérescence donne lieu à un déficit dopaminergique qui s'exprime par ces trois troubles moteurs. Dans l'absence de l'évidence histopathologique, les caractéristiques cliniques déterminent l'appartenance à cette maladie.

Actuellement, le traitement de la maladie de Parkinson se fait entre autre par l'utilisation de substances dopaminergiques, en particulier de la l-DOPA, associée éventuellement à un inhibiteur de la l-DOPA décarboxylase comme la carbidopa, pour éviter les effets secondaires périphériques de la l-DOPA sur le système cardiovasculaire, et ainsi optimiser ses effets centraux.

Cette thérapeutique compense les trop faibles taux cérébraux endogènes de dopamine et améliore la symptomatologie de la maladie, sans pour cela en soigner la cause. Elle possède des inconvénients majeurs tels que dyskinésies tardives, des effets indésirables d'ordre gastro-intestinaux et cardiovasculaires. De plus, il existe un épuisement de l'effet. La l-DOPA n'arrête pas la progression de la maladie puisque après arrêt du traitement, les symptômes réapparaissent immédiatement. Un besoin thérapeutique s'attachant à la récupération de la dégénérescence neuronale existe.

Il est connu que l'Efaroxan, 2-(2-éthyl-2,3 dihydrobenzofuranyl)-2-imidazoline, possède des propriétés antagonistes sur les récepteurs α₂ adrénergiques. (voir par exemple G. Joly et al., Arch. Int. Pharmacodyn. Ther., vol. 277 No. 2, 1985, pages 180-191).

Ce composé est décrit dans le brevet GB-2 102 422 par sa structure chimique dans une formule générale, son procédé de synthèse, ses formulations pharmaceutiques et son application thérapeutique en tant que médicament antidéprésseur et antimigraineux.

Ce composé est également décrit dans le brevet WO 92/05171 où est mis en évidence l'action de l'énantiomère levogyre pour traiter le diabète, comme agent bloqueur des canaux potassiques.

Différentes études ont également été menées sur des singes ou des rats pour évaluer l'action de différents composés sur des symptômes analogues à ceux de la maladie de Parkinson, tels que les "symptômes" induits par la réserpine chez le rat (F.C. COLPAERT, Neuropharmacology, 26, 1431, 1987) ou par la neurotoxine MPTP (F.C. COLPAERT et al., Brain Res. Bull, 26, 627, 1991), ou encore les symptômes associés chez l'homme avec une autre maladie extrapyramidale : la paralysie supranucléaire progressive (J. GHIKA et al., Neurologie, 41, 986, 1991).

Les composés étudiés ont été choisis parmi différents agonistes de la dopamine, anticholinergiques, agonistes de la 5-HT, de l'histamine et certains agonistes ou antagonistes des récepteursα-adrénergiques, parmi lesquels l'idazoxan.

Toutefois, ces études générales ont porté sur des maladies induites qui, bien que possédant un certain nombre de similitudes au niveau de quelque symptômes, sont différentes et s'en distinguent, notamment la paralysie supranucléaire du fait qu'elle n'affecte que les neurones intrinsèques du néostratium et que les traitements dopaminergiques (l-DOPA), n'induisent pas d'améliorations.

Or, il a été trouvé d'une manière inattendue, que l'utilisation de l'Efaroxan ou l'un de ses dérivés permettait non seulement de traiter la maladie de Parkinson, mais également d'observer une persistance des améliorations obtenues, même après l'arrêt du traitement.

La présente invention concerne donc l'utilisation de l'Efaroxan et ses dérivés pour la préparation d'un médicament destiné au traitement de la maladie de Parkinson, et de son évolution.

Par Efaroxan et ses dérivés, on entend le composé de formule I 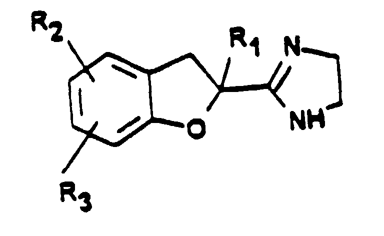 dans laquelle
R₁ représente un atome d'hydrogène, un groupe alkyle linéaire ou ramifié en C₁-C₆,
R₂ représente un atome d'hydrogène, un groupe méthyl, chloro, bromo ou fluoro,
R₃ représente un atome d'hydrogène, un groupe méthyl, hydroxy, méthoxy, fluoro, chloro ou bromo,
sous sa forme racémique et sous la forme optiquement active de ses deux énantiomères ainsi que les sels thérapeutiquement acceptables.

De manière préférentielle, R₂ et R₃ représentent un atome d'hydrogène, et R₁ représente un groupe éthyle ou un groupe n-propyle.

### ETUDE PHARMACOLOGIQUE

Une étude pharmacologique a été menée chez le rat selon le test d'Ungerstedt (Acta Phys. Scand. (1971) 367,67). La projection dopaminergique nigro-striée a été détruite par injection de la 6-OH dopamine et la rotation observée après administration de l'amphétamine. Des antagonistes des récepteurs α2, administrés juste avant l'amphétamine augmentaient les rotations de façon dose-dépendante (voir tableau 1). Ces résultats montrent une potentialisation de l'activité dopaminergique de l'amphétamine par stimulation de la voie noradrénergique.

**Tableau 1**

| **Produit** | **Doses (mg/kg ip)** | **% effet** |
|---|---|---|
| **Methoxy Idazoxan (RX 821002)** | 0,16 | + 94 |
| | 0,63 | + 83 |
| **Efaroxan** | 0,16 | + 43 |
| | 0,63 | + 89 |

Une étude de microdialyse a été effectuée suivant les conditions de Lategan et al., 1992 (A.J. LATEGAN. M.R. MARIEN, F.C. COLPAERT, Life Science 50, 995, 51992). Une sonde de microdialyse a été implantée dans le striatum du rat sous anesthésie. Le taux de dopamine dans le dialysat a été analysé par HPLC. L'administration des antagonistes des récepteurs α2 augmentait le taux de dopamine (tableau 2) suggérant une augmentation de la libération de dopamine par stimulation de la voie dopaminergique.

**Tableau 2**

| **Produit** | **Doses (mg/kg ip)** | **% effet sur le taux de DA** |
|---|---|---|
| **ethoxy Idazoxan (RX 811059)** | 10 | + 200 |
| **Efaroxan** | 10 | + 250 |

L'augmentation des taux de DA au niveau du striatum ainsi que les tests comportementaux provoqués par des substances α₂ antagonistes conduisent à appliquer cette propriété pour obtenir des médicaments utiles dans le traitement de la maladie de Parkinson.

### ETUDE GALENIQUE

Les compositions pharmaceutiques sont administrées par voie orale sous forme de gélules ou de comprimés dosés de 1 à 100 mg de principe actif, plus particulièrement de 2, 5 et 20 mg par gélule, ou par voie intraveineuse sous forme de soluté injectable dosée de 0,1 à 10 mg d'Efaroxan.

### ETUDE CLINIQUE

Une étude pilote a été effectuée et 20 patients ont été sélectionnés répondant aux critères idiopathiques de la maladie de Parkinson. Deux groupes ont été faits, groupe I traité par l'Efaroxan et groupe II sous placebo. La symptomatologie est observée avant traitement et à l'issue de 1 mois de traitement (voir tableau 3). La posologie a été de 6 à 8 mg par jour (et plus quand bien toléré).

**Tableau 3**

| | **Avant traitement** | **Après traitement** |
|---|---|---|
| **Groupe I** | ++++ | ++ |
| **Groupe II** | ++++ | ++++ |
| + = gravité des troubles moteurs | | |

Ces résultats montrent que le traitement de la maladie de Parkinson par un α₂ antagoniste comme l'Efaroxan ou ses dérivés, apporte une amélioration dans la symptomatologie et est susceptible d'influer favorablement sur le rythme de progression de la maladie.

## Revendications

1. Utilisation d'un composé de formule générale I 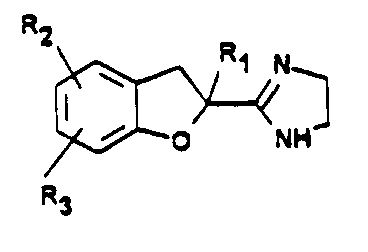 dans laquelle
R1 représente un atome d'hydrogène, un groupe alkyle linéaire ou ramifié en C₁-C₆,
R₂ représente un atome d'hydrogène, un groupe methyl, chloro, bromo ou fluoro,
R₃ représente un atome d'hydrogène, un groupe méthyl, hydroxy, méthoxy, fluoro, chloro ou bromo
et ses sels thérapeutiquement acceptables, son racémique, ou ses isomères optiquement actifs, pour la préparation d'un médicament destiné au traitement de la maladie de Parkinson, et de son évolution.

2. Utilisation selon la revendication 1, caractérisé en ce que R₂ et R₃ représentent un atome d'hydrogène.

3. Utilisation selon l'une des revendications 1 ou 2, caractérisée en ce que R₁ représente un groupe éthyle.

4. Utilisation selon l'une des revendications 1 ou 2, caractérisée en ce que R₁ représente un groupe n-propyle.

## Claims

1. Use of a compound of general formula I 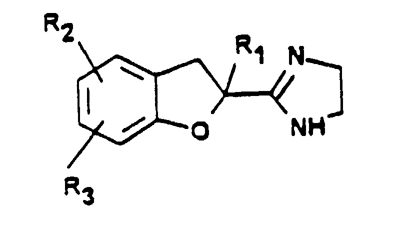 in which
R₁ represents a hydrogen atom or a linear or branched C₁-C₆ alkyl group,
R₂ represents a hydrogen atom or a methyl, chloro, bromo or fluoro group,
R₃ represents a hydrogen atom or a methyl, hydroxyl, methoxy, fluoro, chloro or bromo group,
and its therapeutically acceptable salts, its racemate or its optically active isomers, for the preparation of a medicinal product intended for the treatment of Parkinson's disease and of its progression.

2. Use according to Claim 1, characterized in that R₂ and R₃ represent a hydrogen atom.

3. Use according to either of Claims 1 and 2, characterized in that R₁ represents an ethyl group.

4. Use according to either of Claims 1 and 2, characterized in that R₁ represents an n-propyl group.

## Patentansprüche

1. Verwendung einer Verbindung der allgemeinen Formel I 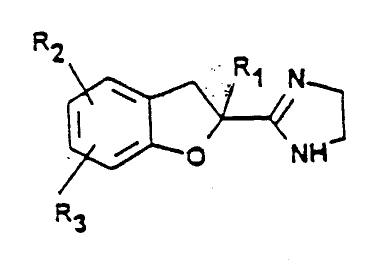
wobei R₁ ein Wasserstoffatom, eine lineare oder verzweigte (C₁-C₆)Alkylgruppe darstellt,
R₂ ein Wasserstoffatom, eine Methyl-, Chloro-, Bromo- oder Fluorogruppe darstellt,
R₃ ein Wasserstoffatom, eine Methyl-, Hydroxy-, Methoxy-, Fluoro-, Chloro- oder Bromogruppe darstellt
und ihrer therapeutisch verträglichen Salze, ihres Racemats, oder ihrer optisch aktiven Isomere zur Herstellung eines Medikaments zur Behandlung der Parkinson-Krankheit und ihrer Entwicklung.

2. Verwendung nach Anspruch 1, dadurch charakterisiert, daß R₂ und R₃ ein Wasserstoffatom darstellen.

3. Verwendung nach einem der Ansprüche 1 oder 2, dadurch charakterisiert, daß R₁ eine Ethylgruppe darstellt.

4. Verwendung nach einem der Ansprüche 1 oder 2, dadurch charakterisiert, daß R₁ eine n-Propylgruppe darstellt.
